# EUROPEAN PATENT APPLICATION

(11) **EP 4 155 311 A1**
(43) Date of publication of application: **29.03.2023**
(21) Application number: 21198816.7
(22) Date of filing: 24.09.2021
(51) Int. Cl.: C07D 493/08, C07D 519/00, C10M 105/22

(54) **SURFACTANTS FROM BIO-BASED FEEDSTOCK**

(71) Applicant: NEDERLANDSE ORGANISATIE VOOR TOEGEPAST- NATUURWETENSCHAPPELIJK ONDERZOEK TNO, 2595 DA 's-Gravenhage (NL)
(72) Inventor: CROCKATT, Marc, 2595 DA 's-Gravenhage (NL); KÖNST, Paul Mathijs, 2595 DA 's-Gravenhage (NL); VAN DER WAAL, Jan Cornelis, 2595 DA 's-Gravenhage (NL)
(74) Representative: V.O.

(57) **Abstract**

The invention is directed to a hydrogenated Diels-Alder adduct according to formula (I) or a salt thereof 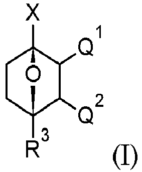 wherein X is is H or an aliphatic moiety that is optionally substituted with one or more heteroatom-comprising substitutents such as hydroxyl,
preferably CH₂OH or H, more preferably H;
R³ is an aliphatic chain comprising at least 6 carbon atoms
Q¹ and Q² together or individually represent one or two polar head groups and comprise one or more hydrophilic moieties.

In a further aspect, the invention is directed to intermediate compounds and methods to prepare the hydrogenated Diels-Alder adduct.

## Description

The invention is in the field of surfactants. In particular, the invention is directed to hydrogenated Diels-Alder adducts that are surfactants. The invention further relates to the method for the preparation of the hydrogenated Diels-Alder adducts, the use thereof as surfactants and formulations comprising the adduct.

Surfactants, or surface-active agents, are chemical compounds that are capable of lowering the surface tension between two liquids or between a gas and a liquid. Surfactants are widely used for a range of applications, such as detergents, emulsifiers, foaming agents and dispersants. Typically, the compounds are organic amphiphilics, *i.e.* compounds with both a hydrophobic tail and a hydrophilic moiety. This combination may allow for a water-soluble component and a water-insoluble (*i.e.* oil-soluble) component. Accordingly, the surfactant may absorb at *i.a.* oil/water interphases and can provide interesting properties for a variety of applications.

It is expected that the market for surfactants undergoes continuous growth in the coming years. The main contributor to the market constitutes detergents and cleaners for households as over 50% of all surfactants typically end up in these products. Currently, the largest production comprises petrochemical-based linear alkylbenzene sulfonates (LAS), with around 4 million tons per year being produced and consumed.

LAS surfactants are typically a mixture of compounds based on a hydrophilic sulfonate moiety attached to a hydrophobic benzene ring which is attached to a hydrophobic alkyl chain. The big advantage of LAS is its tolerance of hard water, which commonly inhibits the functioning of other groups of surfactants. Further, LAS typically has a low critical micelle concentration, low Krafft temperature, fast wetting and good foaming behaviour. LAS is accordingly suitable for broad use in a variety of applications.

However, LAS is fossil-based and has a negative environmental impact. Accordingly, there is a strong desire to provide more sustainable surfactants which have comparable or improved properties compared to LAS.

One method to provide surfactants produced from bio-based chemicals such as sugar-derived furans is described in e.g. WO2017/079718 and by Park et al. (ACS Cent. Sci. 2016, 2, 11, 820-824). Here, oleo-furan surfactants derived from furan and fatty acids are described. By appropriately selecting the fatty acid, the surfactant properties could be altered. Additionally, the surfactants could better tolerate the metal ions contained in hard water, therefore making chelating agents, which are typically added to e.g. detergents, unnecessary.

However, these surfactants are synthesized from furan. While it can be produced as a bio-based molecule, it requires a decarbonylation of furfural, a readily available bio-based platform chemical, thereby decreasing the atom efficiency and typically resulting in a waste stream of carbon monoxide. Additionally, it requires an additional processing step resulting in an increase in cost and inefficiency.

Other bio-based surfactants are described by Kipshagen et al. (Green Chem. 2019, 21, 3882). Here *i.a.* furfural and 5-hydroxymethylfurfural (5-HMF) are used as the basis for the surfactants. In the case of furfural, it is converted into tetrahydrofuran followed by side-chain manipulation and the introduction of a hydrophilic head. In the case of HMF, it is first hydrogenated to 2,5-bis(hydroxymethyl)furan (BHMF) followed by side-chain manipulation and introduction of the hydrophilic head.

5-HMF has also been used as a starting material for surfactants as described in WO2016028845, wherein the final surfactants were prepared by esterification, amination and alkylation to produce non-ionic surfactants. However, these do not mimic LAS.

Other surfactants are described in e.g. WO2021/083642, WO2015/084813 and WO2017/079719.

It is an object of the present inventors to provide a compound that is a surfactant, and which can be obtained from bio-based resources. Additionally, it is an object of the present inventors to prepare the alternative compound with an improved method that overcomes at least part of the above-mentioned drawbacks. The present inventors surprisingly found that such a surfactant can be a hydrogenated Diels-Alder adduct.

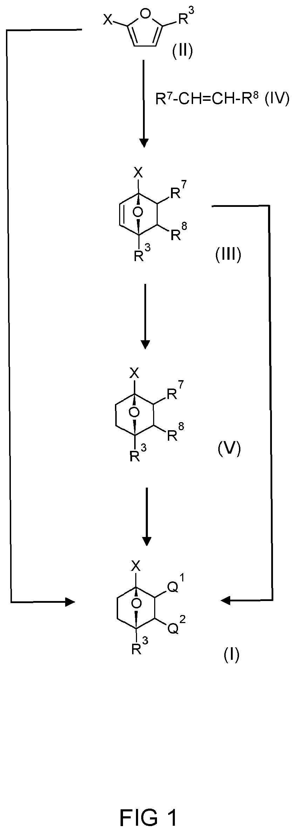

Figure 1 illustrates possible reaction paths according to preferred embodiments of the present invention.

Thus, in a first aspect, the present invention is directed to a hydrogenated Diels-Alder adduct (herein also referred to as "hydrogenated adduct", "adduct" or "Diels-Alder adduct") according to formula (I) or a salt thereof.

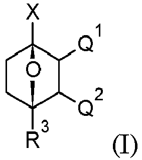

In formula (I), X is H or an aliphatic moiety that is optionally substituted with one or more heteroatom-comprising substituents such as hydroxyl. X may for example be CH₂OH or H. The substituents Q¹ and Q² together or individually represent one or two polar head groups and comprise one or more hydrophilic moieties, while R³ is an aliphatic chain comprising at least 6 carbon atoms. The aliphatic chain provides the hydrophobic tail for the hydrogenated Diels-Alder adduct to function as a surfactant. R³ may desirably be tuned to provide e.g. biodegradability, solubility and other surfactant properties. Surfactant properties may include critical micelle concentration, Krafft temperature, Draves wetting, foam growth rate, foam height and micelle stability. The Diels-Alder adducts according to the present invention may be preferred over aromatized Diels-Alder-derived products as the aromatization step is typically not optimal and includes the loss of an oxygen atom.

Aliphatic chain is herein used to describe a carbon chain that may be linear or branched, linear or cyclic, saturated or unsaturated but not aromatic. Thus, the carbons in the aliphatic chain may be joined by single bonds, one or more carbon pairs may be joined by a double bond and/or one or more carbons may be joined by a triple bond. Also, the chain may comprise one or more non-aromatic carbocyclic moieties. In case the aliphatic chain is branched or cyclic, the main chain (*i.e.* the longest chain of carbon atoms) is considered the backbone chain. Preferably, if the aliphatic chain comprises a certain number of carbon atoms, e.g. at least 6 carbon atoms, it is preferred that the backbone chain comprises said number of carbon atoms, e.g. at least 6 carbon atoms. The branch may also be an aliphatic chain.

The terms polar, apolar, hydrophilic and hydrophobic are commonly used in the field of surfactants. It is commonly known that a surfactant is amphiphilic and comprises hydrophobic (or apolar) and hydrophilic (or polar) moieties. Accordingly, if a compound in accordance with the present invention acts as a surfactant, the moiety of the substituents Q¹, Q², R¹ and/or R² of the compound are considered to be hydrophilic, while the aliphatic chain is considered hydrophobic. In general, hydrophilic is used to describe the capacity of a molecular entity or of a substituent to interact with polar solvents, in particular with water, or with other polar groups and the tendency to mix, be wetted and/or dissolve in water. Typically, hydrophobic is used for moieties that form Van der Waals bonds and minimal to no hydrogen bonds. For instance, aliphatic chains may be considered hydrophobic, while sulfonic and carboxylic acid groups are considered hydrophilic.

In the field of surfactants, various polar head groups are known. In principle, Q¹ and Q² can together or individually represent any of the commonly known polar head groups. The hydrophilic moiety may thus be an ionic moiety, e.g. an anionic moiety, or a non-ionic moiety, in accordance with conventionally known ionic and non-ionic surfactants. Accordingly, suitably, Q¹ and Q² may independently comprise anionic moieties such as sulfate, sulfonate, sulfinate, thiosulfate, carboxylate, phosphate, phosphonate and the like. Preferably, Q¹ and Q² individually or together comprise one or more carboxylic acid moieties, sulfonic acid moieties or combinations thereof.

Examples of suitable non-ionic moieties that Q¹ and Q² may independently comprise or form with the C(O)-group to which they are attached, include poly(ethylene oxide), poly(co-ethylene oxide co-propylene oxide) (also referred to as poloxamers), polyglycosides, isosorbide and its derivatives, 1,4-sorbitane and its derivatives, and the like.

In the adduct according to formula (I), at least one and preferably both of Q¹ and Q² together or individually represent one or two polar head groups. However, in particular embodiments, one of Q¹ and Q² may represent hydrogen.

In preferred embodiments, the hydrogenated Diels-Alder adduct is according to formula (Ia) or a salt thereof 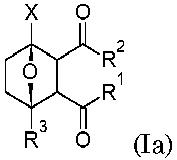
wherein X is as defined for formula I, preferably CH₂OH or H, more preferably H;
R¹ and R² comprise or together form a hydrophilic moiety; and
R³ is an aliphatic chain comprising at least 6 carbon atoms.

Suitably, R¹ and R² may independently comprise or form with the C(O)-group to which they are attached, anionic moieties such as sulfate, sulfonate, sulfinate, thiosulfate, carboxylate, phosphate, phosphonate and the like. Examples of suitable non-ionic moieties that R¹ and R² may independently comprise or form with the C(O)-group to which they are attached, include poly(ethylene oxide), poly(co-ethylene oxide co-propylene oxide) (also referred to as poloxamers), polyglycosides, isosorbide and its derivatives, 1,4-sorbitane and its derivatives, and the like.

Preferably, R¹ and R² are OH such that two hydrophilic groups each form a carboxylic acid (-COOH) or a salt thereof, or R¹ and R² may together form -NR₄-, in accordance with formula Iaa, wherein R⁴ is a moiety comprising a carboxylic acid, a sulfonic acid (SO₃H) or a salt thereof.

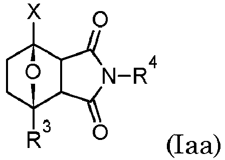

In such cases, a closed 5-membered imide ring is present. R⁴ is an acidic moiety and may for instance be derived from an amino acid or amino sulfonic acid. As such, it may contain an aliphatic chain (e.g. at most 5 carbon atoms), optionally substituted with e.g. one or more carboxylic acids and/or sulfonic acids. It may be appreciated that R⁴ does not detrimentally affect the surfactant properties.

It is most preferred that R¹ and R² are each OH, such that the compound is a di-carboxylic acid. Such Diels-Alder adducts may also advantageously function as chelating agents, thereby limiting the need to add additional chelating agents to formulations wherein the adduct is used, *e.g.* detergents. Additionally, the reaction to form the di-carboxylic acid (*vide infra*) is selective, provides a high yield and includes conventional reactions.

Carboxylic and sulfonic acids readily lose a proton to form a negatively-charged moiety. Accordingly, the hydrogenated Diels-Alder adduct may be present as a salt (*i.e.* with a counter-ion) to balance the negative charge of a deprotonated carboxylic or sulfonic acid. Preferably, the counter ion is an ammonium ion, an alkali ion or alkaline earth metal ion. The counter-ion may for instance be a monovalent cation, such as a sodium (Na⁺) or potassium (K⁺) ion. Nonetheless, it may be appreciated that any suitable counter-ion or combination thereof can be present to balance the charges. A particularly preferred embodiment is a disodium dicarboxylic acid hydrogenated Diels-Alder adduct. It was surprisingly found that such Diels-Alder adducts are relatively stable.

In the hydrogenated Diels-Alder adduct of the present invention, the hydrophobic tail is provided by R³. The hydrophobic tail may suitably be an aliphatic hydrocarbon chain, an aliphatic ether, an aliphatic ester, an aliphatic amide or an aliphatic ketone. Preferably, R³ is selected from the group consisting of: -R^{5'}, -R⁵-O-R⁶ such as -CH₂-O-R⁶, -C(O)-O-R⁶, -R⁵-O-C(O)-R⁶ such as -CH₂-O-C(O)-R⁵, -C(O)-N(R⁶)-R^{6'}, -R⁵-N(R⁶)-C(O)-R^{6'} such as -CH₂-N(R⁶)-C(O)-R^{6'}, -R⁵-C(O)R⁶, and -C(O)-R⁶, wherein R⁵, R^{5'}, R⁶ and R^{6'} are each independently an aliphatic hydrocarbon chain. Most preferably, R³ is R^{5'} or CH₂-O-R⁶. It is typically preferred that the sum of carbon atoms in R⁵, R^{5'}, R⁶ and R^{6'} is at least 6 carbon atoms. Six carbon atoms is roughly considered a minimum amount required to provide suitable hydrophobicity. However, a longer aliphatic chain comprising more carbon atoms may provide more optimal surfactant properties. Accordingly, it is typically preferred that the sum of carbon atoms in R⁵ and R⁶ is at least 8 carbon atoms, such as between 8 to 26 carbon atoms or between 8 and 18 carbon atoms.

A further aspect of the invention is directed to the preparation of the hydrogenated Diels-Alder product. The hydrogenated Diels-Alder product may be prepared through a plurality of steps. Beneficially, the method according to the present invention allows for stabilization of the furan ring while introducing the hydrophilic moiety. A first step may comprise preparing a diene comprising the hydrophobic tail. The diene is preferably a bio-based furanic compound. The furanic compound may for instance be derived from a C₅-sugar, such as furfural, or from a C₆-sugar, such as 5-HMF. The present inventors surprisingly found that furfural can particularly beneficially be used in the present method. Furfural is typically more stable and easier to purify than 5-HMF. Using furfural in the method according to the invention typically leads to the hydrogenated Diels-Alder product wherein R³ is -R^{5'}-CH₂-O-R⁶ and X is H.

Further, advantageously, furfural is a readily available furanic compound as it is for instance currently commercially produced from hemicellulose waste streams from the agricultural industry (e.g. corn, sugar). Moreover, C₅-sugars are typically less expensive than C₆ sugars. Furfural may also be converted to furfuryl alcohol by hydrogenolysis. This process is commercially implemented. Presently, on a yearly basis, 100s of ktons furfuryl alcohol are produced, while HMF is only available on small scale and furan only has bio-based production on a scale of ktons/annum. Accordingly, the diene may also be furfuryl alcohol. Advantageously, the atom efficiency is typically increased when furfuryl alcohol or furfural is used instead of a furan. Additionally, by using furfural or furfuryl alcohol, fewer synthesis steps are typically required compared to furan as starting material, thus reducing costs and increasing efficiency.

The preparation of the diene can be performed by a variety of methods. One method may for instance comprise an aldol reaction of a furanic compound with an aldehyde, ester or ketone. It may be appreciated that the aldehyde and/or ketone can also be formed *in situ* by oxidation of an alcohol. Aldol reactions are well-known in the art. Typically the aldehyde, ester or ketone comprises a hydrophobic tail that determines R³. For instance, a fatty acid-derived aldehyde, ester or ketone may be used.

Alternatively, the diene may also be prepared by side-chain manipulation (e.g. etherification, alkylation, esterification) of furfuryl alcohol to introduce R³. Independent of the preparation method, the diene is typically according to formula (II).

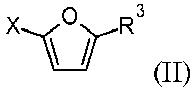

The diene may be subjected to a Diels-Alder reaction with a dienophile to obtain an unsaturated bicyclic ether. The Diels-Alder reaction can for example be carried out using conditions disclosed in WO 2019/070123 and references cited therein (which are incorporated herein in their entirety).

Suitable dienophiles are according to formula (IV).

R⁷―CH=CH―R⁸ (IV)

In formula (IV), R⁷ and R⁸ are independently selected from the group consisting of hydrogen, -CN, CHO, SO₃R⁹, C(O)N(R⁹)₂ and COOR⁹. Notably, at most one of R⁷ and R⁸ can be hydrogen, as one or both of R⁷ and R⁸ represent of must be convertible into the polar head groups of the adduct. Of R⁷ and R⁸, R⁹ is or are independently selected from the group consisting of hydrogen and aliphatic chains comprising 1 to 8 carbon atoms, optionally substituted with one or more, preferably hydrophilic, substituents. These substituents are preferably one or more carboxylic or sulfonic acid groups.

Accordingly, the unsaturated bicyclic ether is preferably according to formula (III), wherein R⁷ and R⁸ are as defined for formula (IV).

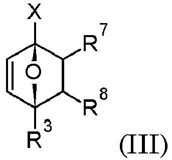

In a particular embodiment, R⁷ and R⁸ together from a -C(O)-O-(O)C- group such that the dienophile is maleic anhydride, which will result in the unsaturated bicyclic ether is preferably according to formula (IIIc). 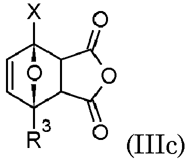 R⁷ and R⁸ in the dienophile may alternatively together form a - C(O)-NR¹⁰-C(O)- group, as illustrated by formula IVaa, wherein R¹⁰ represents hydrogen or an aliphatic moiety comprising one or more carboxylic acids, and/or sulfonic acids.

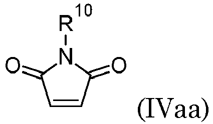

Such a dienophile may for instance be formed by the reaction of maleic anhydride with an amino acid derivative or an amino sulfonic acid derivative. It may be appreciated that amino sulfonic acid refers to a compound comprising both an amine functionality and a sulfonic acid or derivative thereof, wherein these functional groups may be separated by one or more carbon or heteroatoms. Accordingly, the dienophile may specifically be according to formula (IVab) or (IVac), wherein R¹¹ is the side group of the amino acid or amino sulfonic acid, and preferably selected from the group consisting of hydrogen and aliphatic chains comprising 1 to 8 carbon atoms, optionally substituted with one or more, preferably hydrophilic, substituents such as carboxylic acids, sulfonic acids or combinations thereof, and wherein A is alkylene, preferably CH or CH₂-CH.

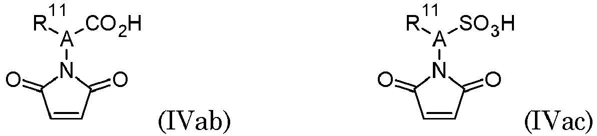

Embodiments wherein the dienophile is of formula (IVaa) typically results in the hydrogenated Diels-Alder product of formula (Iaa), via unsaturated bicyclic ether according to formula (IIIaa). It may be appreciated that R¹⁰ in those embodiments may be equal to R⁴, or that R¹⁰ may be converted into R⁴.

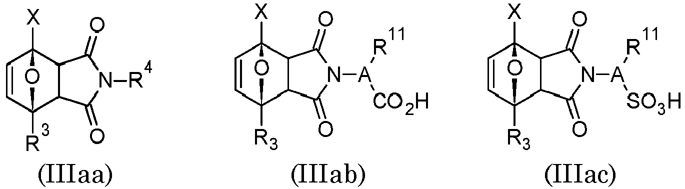

Similarly, embodiments wherein the dienophile is of formula (IVab) or (IVac) typically results respectively in the hydrogenated Diels-Alder products of formula (Iab) or (Iac), via unsaturated bicyclic ether according to formula (IIIab) or (IIIac), respectively, wherein A and R¹¹ may be as defined for formulae (IVab) and (IVac).

Further, the hydrogenated Diels-Alder product of formula Ia may be obtained by reacting the unsaturated bicyclic ether (III) or a hydrogenated saturated bicyclic ether (V) (*vide infra*) with an amine, amino acid or amino sulfonic acid such an H₂N-R⁴, R¹¹-A-CO₂H or R¹¹-A-SO₃H respectively.

The Diels-Alder reaction may provide endo products, exo products or a combination thereof. Accordingly, any stereochemistry indicated herein typically refers to relative stereochemistry.

The unsaturated bicyclic ether (III) may further be subjected to hydrogenation to obtain a saturated bicyclic ether (V).

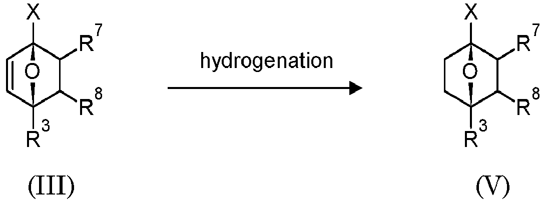

Hydrogenation is a well-known reaction in the art and reduces the double bond of compound (III) to a single bond as illustrated in compound (V). It is typically performed in the presence of a catalyst such as a catalyst surface comprising palladium, nickel and/or platinum. Hydrogenation may be performed *in situ* or *ex situ* with Diels-Alder reaction. Particularly suitable reaction conditions for the Diels-Alder reaction and hydrogenation are for instance disclosed in WO2016/099274, which is incorporated herein in its entirety.

The substituents R⁷ and/or R⁸ of formula (V) may correspond to the polar head groups Q¹ and/or Q² of the surfactant of formula (I), for instance when the Diels-Alder reaction is carried out with a dienophile according to any of formulae (IVaa), (IVab) or (IVac), in which case no or only partial modification of R⁷ and/or R⁸ is required to convert the saturated bicyclic ether (V) into the surfactant of formula (I). In other embodiments, however, R⁷ and R⁸ can be converted into Q¹ and Q². For example, in the embodiments wherein R⁷ and/or R⁸ represent CN, SO₃-alkyl or CO₂-alkyl, or together form the -C(O)-O-(O)C- group, hydrolyzing R⁷ and R⁸ may be preferred to provide hydrophilic moieties.

Thus, depending on the desired adduct, the saturated bicyclic ether (V) may for example be subjected to hydrolysis or condensation with a suitable nucleophile to form the desired Q¹ and Q² groups with hydrophilic moieties.

For instance, in a preferred embodiment, the saturated bicyclic ether according to formula Vc is subjected to hydrolysis, which can open the 5-membered ring and form the adduct comprising two carboxylic acids as hydrophilic moieties.

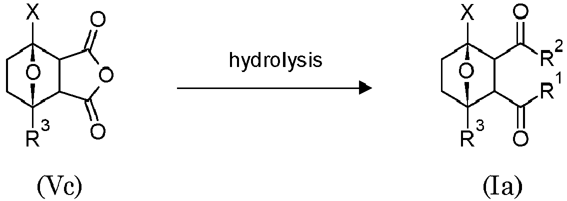

Hydrolysis may be performed using neutral, acidic or basic aqueous conditions to form the adduct of formula (Ia) wherein R¹ and R² are both OH, or salts thereof.

Similarly, the reaction of compound (Vc) to form a non-ionic surfactant of formula (Ia) can be carried out using other nucleophiles, e.g. poly(ethylene oxide), poly(co-ethylene oxide co-propylene oxide) (also referred to as poloxamers), polyglycosides, isosobide and its derivatives, 1,4-sorbitane and its derivatives.

In a preferred embodiment, the reaction of compound (Vc) can be carried out using an amine H₂N-R⁴ to form the adduct of formula (Iaa), wherein R⁴ may be as defined for formula (IVaa).

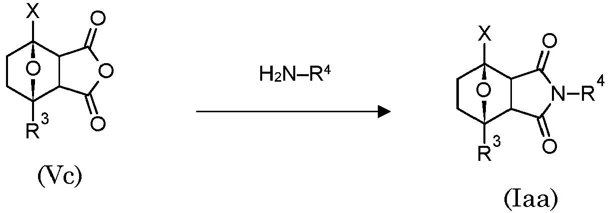

In a more preferred embodiment, the reaction of compound (Vc) can be carried out using amino acids or amino sulfonic acids, preferably α- or β- amino acids or amino sulfonic acids of formulae R¹¹-A-CO₂ or R¹¹-A-SO₃ to yield the adduct comprising the imide ring of formula (Iab) or (Iac), wherein A and R¹¹ may be as defined for formulae (IVab) and (IVac).

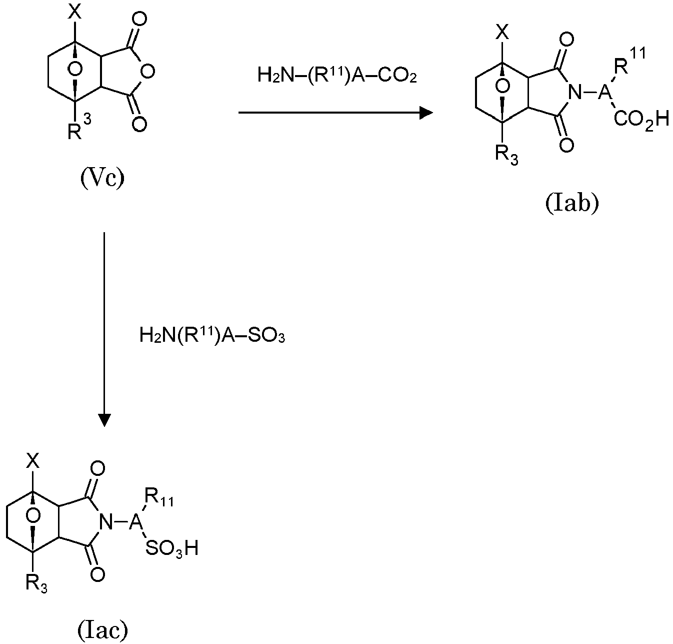

Optionally, adducts of formulae Iaa, Iab and Iac can be obtained by intermediately hydrolyzing compound (Vc) before its reaction with said amino acids or amino sulfonic acids.

It may be appreciated that one or more reactions as described herein may be combined in one process step. Accordingly, for example, two or more of the Diels-Alder, hydrogenation, condensation and/or hydrolysation reactions as described herein may be combined. For example, the synthesis comprising the Diels-Alder reaction, hydrogenation and hydrolysis starting from the diene to the adduct may be combined as a hydrogenative Diels-Alder reaction to directly prepare the adduct comprising two carboxylic acids. Similarly, the Diels-Alder reaction may be performed as a single-step followed by a combination reaction comprising hydrogenation and hydrolysis. For the adduct comprising a closed 5-membered ring wherein R¹ and R² together form ―NR_{4―}, the Diels-Alder reaction may be performed using a dienophile according to formula (IV) wherein R⁷ and R⁸ together form a -C(O)-NR¹⁰-C(O)- group, followed by hydrogenation. Figure 1 schematically illustrates the possible reaction paths.

The Diels-Alder adduct or a salt thereof according to formula (I) can advantageously be used as a surfactant as it comprises a hydrophilic moiety and a hydrophobic tail. The hydrophilic moiety and hydrophobic tail may be tuned to provide the optimal surfactant properties. The optimal properties may depend on the final application. For instance, a more hydrophobic Diels-Alder adduct is preferred for applications such as a water in oil emulsifier. A more hydrophilic hydrogenated adduct may be preferred as a detergent or as an oil in water emulsifier. Further, typically the length and/or branching of R³ may be altered to provide a more or less biodegradable surfactant.

The invention is further directed to a formulation comprising the Diels-Alder adduct according to formula (I) or a salt thereof. Such formulations may include cleaning products, such as household-care products, such as detergents and dishwashing liquid and personal care products such as soap and shampoos. The formulations may further include products such as motor oils, paints, inks, agrochemicals, textiles, biological formulations and firefighting products. It may be appreciated that this list is non-exhaustive.

For the purpose of clarity and a concise description, features are described herein as part of the same or separate embodiments, however, it will be appreciated that the scope of the invention may include embodiments having combinations of all or some of the features described.

The invention may further be illustrated by the following nonlimiting examples.

### Example 1

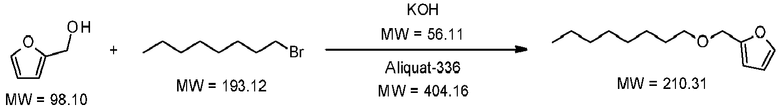

A reactor was charged with potassium hydroxide (35 g) and Aliquat-336 (16.48 g). The mixture was stirred and furfuryl alcohol (40 g) was added. Following this, decyl bromide (100.0 g) was added. The mixture was heated to 80°C for 120 minutes the cooled to room temperature. The mixture was partitioned between water (500 ml) and MTBE (500 ml). The organic phase was dried (Na₂SO₄), filtered and concentrated to yield and oil. This was purified by vacuum distillation to yield octyl-furfuryl ether (91.18 g, 106%). Analysis by NMR & GCMS confirmed the desired product containing residual octyl bromide.

### Example 2

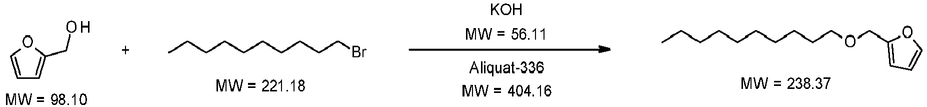

A reactor was charged with potassium hydroxide (35 g) and Aliquat-336 (16.48 g). The mixture was stirred and furfuryl alcohol (40g) was added. Following this, decyl bromide (114.54 g) was added. The mixture was heated to 80 °C for 120 minutes the cooled to room temperature. The mixture was partitioned between water (500 ml) and MTBE (500 ml). The organic phase was dried (Na₂SO₄), filtered and concentrated to yield and oil. This was purified by vacuum distillation to yield decyl-furfuryl ether (92.4 g, 97%). Analysis by NMR & GCMS confirmed the desired product.

### Example 3

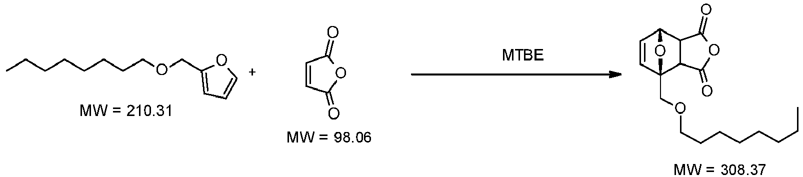

A reactor was charged with maleic anhydride (28.91 g) and MTBE (50 ml). The mixture was heated to 40 °C with stirring and then octyl-furfuryl ether (62.0 g) was added dropwise. The heating was removed and the mixture was allowed to cool to room temperature and was seeded with a small amount of product. This caused further solids to precipitate. The mixture stirred for 30 minutes then the solid was then isolated by filtration. The solid was washed with ice cold MTBE (40 ml) then dried to yield octoxymethyl-Diels-Alder adduct as a solid (48.5 g. 53%). Analysis by NMR confirmed the desired product.

### Example 4

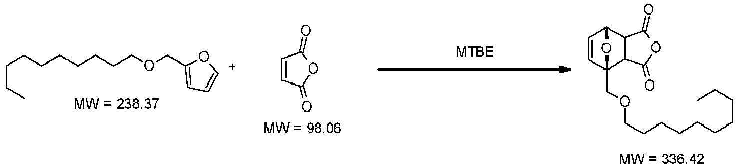

A reactor was charged with maleic anhydride (25.02 g) and MTBE (75 ml). The mixture was heated to 40 °C with stirring and then decyl-furfuryl ether (90.0 g) was added dropwise. The heating was removed and the mixture was allowed to cool to room temperature and was seeded with a small amount of product. This caused further solids to precipitate. The mixture stirred for 30 minutes then the solid was then isolated by filtration. The solid was washed with ice cold MTBE (50 ml) then dried to yield decoxymethyl-Diels-Alder adduct as a solid (104.23 g. 82%). Analysis by NMR confirmed the desired product.

### Example 5

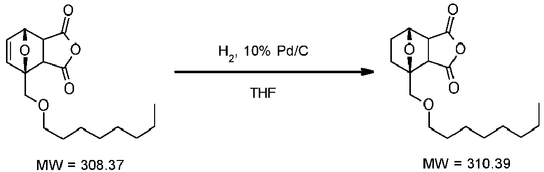

An autoclave was charged with octoxymethyl-Diels-Alder adduct (15 g) and THF (50 ml). 10% Palladium on Carbon (500 mg, 3wt%) was added under a nitrogen atmosphere. The reactor was sealed, flushed twice to 15 bar with N₂, then charged with hydrogen gas to 80 bar. Stirring was then started. When the pressure dropped to 50 bar, hydrogen was re-charged to 80 bar and the mixture left to stir. The mixture was cooled to room temperature then the hydrogen pressure was released and the reactor flush twice to 15 bar with N₂. The mixture was filtered through decalite to remove the catalyst then concentred to an oil which was purified by flash chromatography to yield an oil which precipitated to yield octoxymethyl-H₂-Diels-Alder adduct a solid (9.54 g, 64%). Analysis by NMR confirmed the desired product.

### Example 6

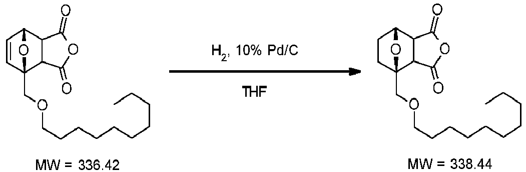

An autoclave was charged with decoxymethyl-Diels-Alder adduct (16.30 g) and THF (50 ml). 10% Palladium on Carbon (500 mg, 3wt%) was added under a nitrogen atmosphere. The reactor was sealed, flushed twice to 15 bar with N₂, then charged with hydrogen gas to 80 bar. Stirring was then started. When the pressure dropped to 50 bar, hydrogen was re-charged to 80 bar and the mixture left to stir. The mixture was cooled to room temperature then the hydrogen pressure was released and the reactor flush twice to 15 bar with N₂. The mixture was filtered through decalite to remove the catalyst then concentred to an oil which precipitated to yield decoxymethyl-H₂-Diels-Alder adduct a solid (16.74 g, 102%). Analysis by NMR confirmed the desired product with some residual solvent present.

### Example 7

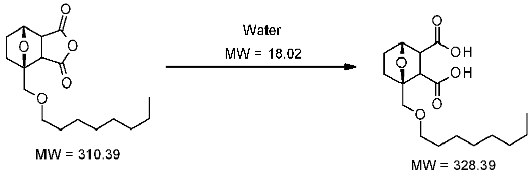

A reactor was charged with octoxymethyl-H₂-Diels-Alder adduct (8.70 g) and water (400 ml). The mixture was heated to 95°C with stirring for two hours then allowed to cool to room temperature with stirring. This resulted in precipitation of a solid. The solid was then isolated by filtration and washed with a minimum amount of ice cold water. The solid was dried to yield octoxymethyl-H₂-Diels-Alder adduct in the di-acid form as a solid (8.10 g. 88%). Analysis by NMR & LCMS confirmed the desired product.

### Example 8

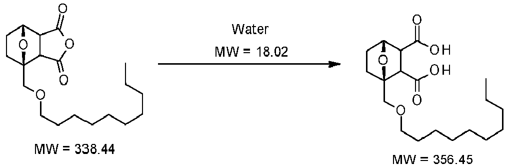

A reactor was charged with decoxymethyl-H₂-Diels-Alder adduct (8.20 g) and water (400 ml). The mixture was heated to 95°C with stirring for two hours then allowed to cool to room temperature with stirring. This resulted in precipitation of a solid. The solid was then isolated by filtration and washed with a minimum amount of ice cold water. The solid was dried to yield decoxymethyl-H₂-Diels-Alder adduct in the di-acid form as a solid (7.85 g. 91%). Analysis by NMR & LCMS confirmed the desired product.

### Example 9

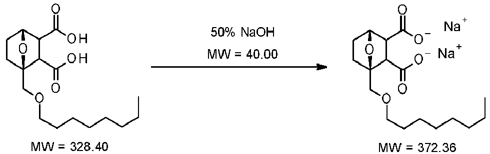

A reactor was charged with octoxymethyl-H₂-Diels-Alder adduct in the di-acid form (1.781 g) and water (40 ml). To this was charged 50% aqueous sodium hydroxide (275 µL). The mixture was heated to 90°C with stirring to achieve a clear solution then was concentrated to -1/5 of the original volume. The mixture was allowed to cool to room temperature with stirring, resulting in precipitation of a solid. The solid was then isolated by filtration and washed with a minimum amount of ice cold water. The solid was dried to yield decoxymethyl-H₂-Diels-Alder adduct in the di-sodium form as a solid (1.41 g. 72%). Analysis by NMR & LCMS confirmed the desired product.

### Example 10

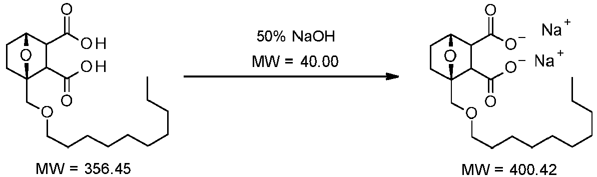

A reactor was charged with decoxymethyl-H₂-Diels-Alder adduct in the di-acid form (1.773 g) and water (40 ml). To this was charged 50% aqueous sodium hydroxide (262 µL). The mixture was heated to 90°C with stirring to achieve a clear solution then was concentrated to ~1/5 of the original volume. The mixture was allowed to cool to room temperature with stirring, resulting in precipitation of a solid. The solid was then isolated by filtration and washed with a minimum amount of ice cold water. The solid was dried to yield decoxymethyl-H₂-Diels-Alder adduct in the di-sodium form as a solid (1.96 g. 98%). Analysis by NMR & LCMS confirmed the desired product.

### Example 11

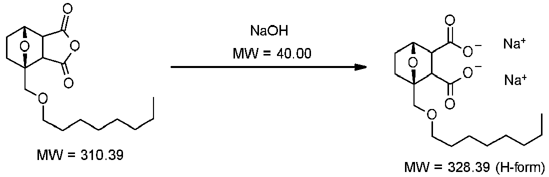

A reactor was charged with octoxymethyl-H₂-Diels-Alder adduct (800 mg), THF (3 ml) and water (3 ml). To this was charged 50% aqueous sodium hydroxide (1144 µL). The THF was removed by evaporation and a solid precipitated. The solid was isolated by filtration and washed with a minimum amount of ice cold water. The solid was dried to yield octoxymethyl-H₂-Diels-Alder adduct in the di-sodium form as a solid (812 mg. 85%). Analysis by NMR & LCMS confirmed the desired product.

## Claims

1. A hydrogenated Diels-Alder adduct according to formula (I) or a salt thereof 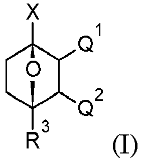
wherein X is H or an aliphatic moiety that is optionally substituted with one or more heteroatom-comprising substitutents such as hydroxyl, preferably CH₂OH or H, more preferably H;
R³ is an aliphatic chain comprising at least 6 carbon atoms Q¹ and Q² together or individually represent one or two polar head groups and comprise one or more hydrophilic moieties.

2. The hydrogenated Diels-Alder adduct according to claim 1, wherein one or both of Q¹ and Q² together or individually comprise one or more carboxylic acid moieties, sulfonic acid moieties, salts thereof and/or combinations thereof.

3. The hydrogenated Diels-Alder adduct according to any of claims 1-2, wherein said is according formula (Ia) or a salt thereof 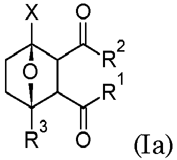
wherein X is H or an aliphatic moiety that is optionally substituted with one or more heteroatom-comprising substitutents such as hydroxyl, preferably CH₂OH or H, more preferably H;
R¹ and R² comprise or together form a hydrophilic moiety; and
R³ is an aliphatic chain comprising at least 6 carbon atoms.

4. The hydrogenated Diels-Alder adduct or a salt thereof according to the previous claim, wherein R¹ and R² are each OH or together form ―NR_{4―}, wherein R⁴ is a moiety comprising a carboxylic acid or a sulfonic acid.

5. The hydrogenated Diels-Alder adduct or a salt thereof according to any of the previous claims, wherein the sum of carbon atoms in R³ is at least 8 carbon atoms, preferably between 8 to 26 carbon atoms, more preferably between 8 and 18 carbon atoms.

6. The hydrogenated Diels-Alder adduct or a salt thereof according to any of the previous claims, wherein R³ is selected from the group consisting of -R⁵', -R⁵-O-R⁶ such as -CH₂-O-R⁶, -C(O)-O-R⁶, -R⁵-O-C(O)-R⁶ such as -CH₂-O-C(O)-R⁵, -C(O)-N(R⁶)-R^{6'}, -R⁵-N(R⁶)-C(O)-R^{6'} such as -CH₂-N(R⁶)-C(O)-R^{6'}, -R⁵-C(O)R⁶, and -C(O)-R⁶, wherein R⁵, R^{5'}, R⁶ and R^{6'} are each independently an aliphatic hydrocarbon chain and wherein the sum of carbon atoms in R⁵, R^{5'}, R⁶ and R^{6'} is at least 6 carbon atoms, preferably at least 8 carbon atoms, more preferably between 8 to 26 carbon atoms, most preferably between 8 and 18 carbon atoms.

7. An unsaturated bicyclic ether according to formula (III), preferably according to any of formulae (IIIaa), (IIIab), (IIIac) and (IIIc), suitable for use in the preparation of the hydrogenated Diels-Alder adduct or a salt thereof according to any of the previous claims 1-4, 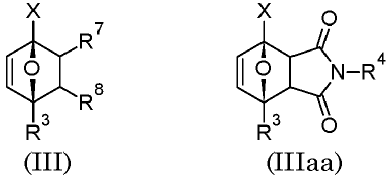 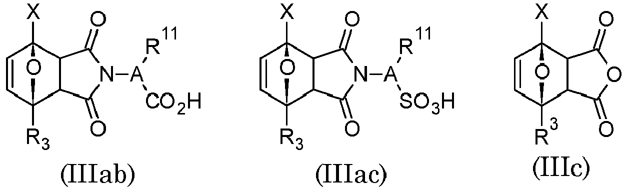
wherein X is H or an aliphatic moiety that is optionally substituted with one or more heteroatom-comprising substitutents such as hydroxyl, preferably CH₂OH or H, more preferably H,
R⁷ and R⁸ are independently selected from the group consisting of hydrogen, -CN, CHO, SO₃R⁹, C(O)N(R⁹)₂ and COOR⁹, provided that at most one of R⁷ and R⁸ is hydrogen, wherein R⁹ is or are independently selected from the group consisting of hydrogen and aliphatic chains comprising 1 to 8 carbon atoms, optionally substituted with one or more, preferably hydrophilic, substituents such a one or more carboxylic or sulfonic acid groups, or wherein R⁷ and R⁸ together form a -C(O)-O-(O)C- group such that the dienophile is maleic anhydride, or wherein R⁷ and R⁸ together form a -C(O)-NR¹⁰-C(O)- group, wherein R¹⁰ represents hydrogen or an aliphatic moiety comprising a carboxylic acid and/or a sulfonic acid;
R³ is an aliphatic chain comprising at least 6 carbon atoms, preferably wherein R³ is selected from the group consisting of: of -R⁵', -R⁵-O-R⁶ such as - CH₂-O-R⁶, -C(O)-O-R⁶, -R⁵-O-C(O)-R⁶ such as -CH₂-O-C(O)-R⁵, -C(O)-N(R⁶)-R^{6'}, -R⁵-N(R⁶)-C(O)-R⁶' such as -CH₂-N(R⁶)-C(O)-R^{6'}, -R⁵-C(O)R⁶, and -C(O)-R⁶, preferably -CH₂-O-R⁶, wherein R⁵, R^{5'}, R⁶ and R^{6'} are each independently an aliphatic chain and wherein the sum of carbon atoms in R⁵, R^{5'}, R⁶ and R^{6'} is at least 6 carbon atoms;
R⁴ is an acidic moiety, preferably comprising a carboxylic acid or a sulfonic acid (SO₃H); and
R¹¹ is the side group of the amino acid or amino sulfonic acid, and preferably selected from the group consisting of hydrogen and aliphatic chains comprising 1 to 8 carbon atoms, optionally substituted with one or more, preferably hydrophilic, substituents.

8. Method for the preparation of an unsaturated bicyclic ether of formula (III), wherein the method comprises reacting a diene according to formula II with a dienophile to form the unsaturated bicyclic ether (III), 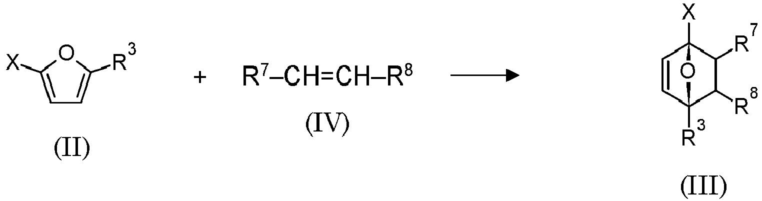
wherein X is is H or an aliphatic moiety that is optionally substituted with one or more heteroatom-comprising substitutents such as hydroxyl, preferably CH₂OH or H, more preferably H,
R³ is an aliphatic chain comprising at least 6 carbon atoms, preferably wherein R³ is selected from the group consisting of: of -R⁵', -R⁵-O-R⁶ such as - CH₂-O-R⁶, -C(O)-O-R⁶, -R⁵-O-C(O)-R⁶ such as -CH₂-O-C(O)-R⁵, -C(O)-N(R⁶)-R^{6'}, -R⁵-N(R⁶)-C(O)-R^{6'} such as -CH₂-N(R⁶)-C(O)-R^{6'} , -R⁵-C(O)R⁶, and -C(O)-R⁶, wherein R⁵, R^{5'}, R⁶ and R^{6'} are each independently an aliphatic chain and wherein the sum of carbon atoms in R⁵, R^{5'}, R⁶ and R^{6'} is at least 6 carbon atoms; and
R⁷ and R⁸ are independently selected from the group consisting of hydrogen, -CN, CHO, SO₃R⁹, C(O)N(R⁹)₂ and COOR⁹, provided that at most one of R⁷ and R⁸ is hydrogen, wherein R⁹ is or are independently selected from the group consisting of hydrogen and aliphatic chains comprising 1 to 8 carbon atoms, optionally substituted with one or more, preferably hydrophilic, substituents such a one or more carboxylic or sulfonic acid groups, or wherein R⁷ and R⁸ together form a -C(O)-O-(O)C- group such that the dienophile is maleic anhydride, or wherein R⁷ and R⁸ together form a -C(O)-NR¹⁰-C(O)- group, wherein R¹⁰ represents hydrogen or an aliphatic moiety comprising a carboxylic acid and/or a sulfonic acid.

9. Method according to the previous claim, wherein the dienophile is according to any one of formulae IVaa, IVab and IVac, 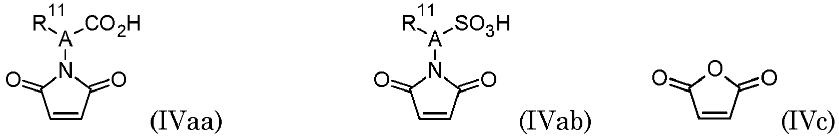 wherein R¹¹ is the side group of the amino acid or amino sulfonic acid, and preferably selected from the group consisting of hydrogen and aliphatic chains comprising 1 to 8 carbon atoms, optionally substituted with one or more, preferably hydrophilic, substituents.

10. Method according to any of claims 8-9, wherein the dienophile is maleic anhydride and the unsaturated bicyclic ether is according to formula (IIIc) 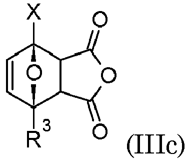 preferably wherein X is H.

11. Method according to any of claims 8-10, wherein the diene is bio-based.

12. Method for the preparation of a saturated bicyclic ether (V), comprising hydrogenating an unsaturated bicyclic ether (III) to obtain the saturated bicyclic ether (V) 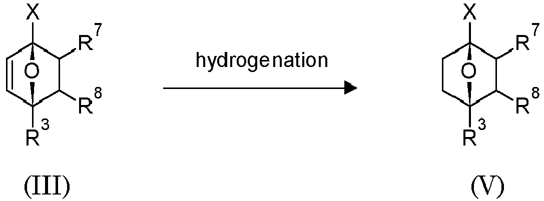 wherein X, R³, R⁷ and R⁸ are as defined in any of claims 5-8.

13. Method for the preparation of the hydrogenated Diels-Alder adduct or a salt thereof according to any of the previous claims 1-6, wherein the method comprises reacting the saturated bicyclic ether of formula (V), wherein X, R³, R⁷ and R⁸ are as defined in claim 8, in a hydrolysis or condensation reaction to form the hydrogenated Diels-Alder adduct or a salt thereof of formula (I) 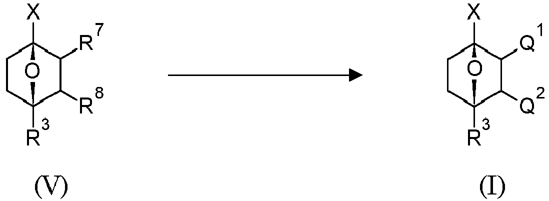 preferably, wherein said method comprises hydrolysis of the saturated bicyclic ether of formula (Vc) to form the hydrogenated Diels-Alder adduct or a salt thereof of formula (Ia) wherein R¹ and R² are OH, or salts thereof, or wherein said method comprises condensation of the saturated bicyclic ether of formula (Vc) with H₂N-R⁴, wherein R⁴ represents is an acidic moiety, preferably comprising a carboxylic acid or a sulfonic acid (SOsH), to form the hydrogenated Diels-Alder adduct or a salt thereof of formula (Iaa).

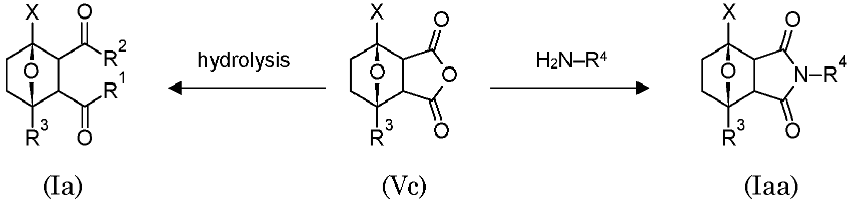

14. Use of the hydrogenated Diels-Alder adduct according to formula (I) or a salt thereof according to any of previous claims 1-6 as a surfactant.

15. Formulation comprising the hydrogenated Diels-Alder adduct according to formula (I) or a salt thereof according to any of the previous claims 1-6.
